# NOUVEAU FASCICULE DE BREVET EUROPEEN

(11) **EP 1 416 958 B2**
(45) Date de publication et mention de la décision concernant l'opposition: **14.03.2012**
(45) Mention de la délivrance du brevet: 28.03.2007
(21) Numéro de dépôt: 02779603.6
(22) Date de dépôt: 31.07.2002
(51) Int. Cl.: A61K 39/295, A61K 47/02

(54) **COMPOSITION VACCINALE COMPRENANT AU MOINS DEUX VALANCES, L'UNE ADJUVANTEE, L'AUTRE PAS**
IMPFSTOFFZUSAMMENSETZUNG, DIE MINDESTENS ZWEI IMMUNOGENE ENTHÄLT, EINES MIT ADJUVANS IMMUNOGEN, EINES OHNE ADJUVANT IMMUNOGEN
VACCINE COMPOSITION COMPRISING AT LEAST TWO VALENCES, ONE ENHANCED WITH ADJUVANT AND NOT THE OTHER

(30) Priorité: 08.08.2001 FR 0110573
(43) Date de publication de la demande: 12.05.2004
(73) Titulaire: Sanofi Pasteur, 69367 Lyon Cedex 07 (FR)
(72) Inventeur: FRANCON, Alain, 69690 Bessenay (FR)
(74) Mandataire: Ayroles, Marie-Pauline
(86) Numéro de dépôt international: PCT/FR2002/002770
(87) Numéro de publication internationale: WO 2003/013600

(56) Documents cités:
- WO-A-96/37222
- WO-A-99/48525
- WO-A2-00/12129
- DUMAS R ET AL: "Safety and immunogenicity of a new inactivated hepatitis A vaccine in concurrent administration with a typhoid fever vaccine or a typhoid fever + yellow fever vaccine." ADVANCES IN THERAPY, vol. 14, no. 4, juillet 1997 (1997-07), pages 160-167, XP008004639 ISSN: 0741-238X
- CORBEL M J: "Reasons for instability of bacterial vaccines." DEVELOPMENTS IN BIOLOGICAL STANDARDIZATION, vol. 87, 1996, pages 113-124, XP008012002 ISSN: 0301-5149
- I.VODOPIJA ET AL: 'Immune response to Hepatitis A vaccine combined or given simultaneously' JOURNAL OF TRAVEL MEDICINE vol. 4, no. 3, 01 Janvier 1997, pages 114 - 117
- C.VAN HOECKE ET AL: 'Concomitant vaccination against Hepatitis A and Typhoid fever' JOURNAL OF TRAVEL MEDICINE vol. 5, no. 3, 01 Janvier 1998, pages 116 - 120
- P.C:FUSCO ET AL: 'Meningococcal vaccine development: a novel approach' EXP.OPIN.INVEST.DRUGS vol. 7, no. 2, 01 Janvier 1998, pages 245 - 252
- J.S.KIM ET AL: 'Determination of saccaride content in pneumococcal polysaccharidesand conjugate vaccines by GC-MSI' ANALYTICAL BIOCHEMISTRY vol. 347, 01 Janvier 2005, pages 262 - 274
- S.K.OBARO ET AL: 'Safety and immunogenicity of pneumococcal conjugate vaccine in combination with diphteria, tetanus toxoid, pertussis and Haemophilus influenza type b conjugate vaccine' PEDIATR.INFECT.DIS. vol. 31, no. 10, 01 Janvier 2002, pages 940 - 946
- S.A.PLOTKIN &E.A.MORTIMER: 'Pneumococcal Vaccines' VACCINES W.D. SAUNDERS CO. 01 Janvier 1988, pages 277 - 279
- DATABASE WWW.IAS,¢.ORG.NZ 01 Janvier 1996 'Tetrammune / Monograph'
- C.V.HOECKE: 'Concomitant vaccination against hepatitis A and typhoid fever' ISTM MEETING MARCH 1997 01 Mars 1997,

## Description

La présente invention a notamment pour objet une composition vaccinale bivalente Hépatite A / Fièvre typhoïde (HA-Vi) stabilisée dans laquelle la valence Vi conserve son pouvoir immunogène pendant au moins 24 mois environ.

L'hépatite A et la fièvre typhoïde sont deux maladies pour lesquelles on dispose déjà d'un vaccin. Elles se rencontrent toutes deux dans des régions du globe où les conditions d'hygiène sont loin d'être optimales. Dans la mesure où les agents infectieux respectifs ont en commun la même voie de transmission (voie orale-fécale) et dans la mesure où les zones endémiques de ces maladies se recouvrent largement, il apparaît intéressant de combiner les deux valences dans un même produit. En particulier, on comprendra aisément que, dans la gamme des vaccins du voyageur, une combinaison HA-Vi soit plus attractive que les deux vaccins monovalents HA et Vi devant être administrés séparément.

Des études ont déjà été réalisées afin de vérifier que les valences HA et Vi étaient compatibles notamment en termes d'innocuité, de pouvoir immunogène et de stabilité. Ces études utilisent des combinaisons HA-Vi préparées à partir des vaccins monovalents existant déjà sur le marché ; elles sont essentiellement au nombre de deux : d'une part, les études conduites en combinant les monovalents Havrix^{™} (HA) et Typherix^{™} produits par SmithKline Beecham Biologicals (Rixensart, Belgique) et d'autre part, les études conduites en combinant les monovalents Avaxim^{™} (HA) et Typhim Vᵢ^{™} (Vi) produits par Aventis Pasteur (Lyon, France).

Dans les deux séries d'études, le vaccin monovalent HA est constitué par du virus dé l'hépatite A inactivé et adsorbé sur de l'hydroxyde d'aluminium. De même, le vaccin monovalent fièvre typhoïde est constitué par du polysaccharide de la capsule de *Salmonella typhi* qui reste non adjuvanté. Enfin, les combinaisons bivalentes sont produites de manière identique, en mélangeant simplement les vaccins monovalents correspondants (ces vaccins monovalents sont commercialisés sous forme liquide). Ainsi, une dose d'Avaxim^{™} et une dose de Typhim Vi^{™} sont mélangées ensemble pour donner une dose de la combinaison bivalente HA-Vi.

Les deux séries d'études ont été réalisées avec des combinaisons constituées moins de vingt mois avant que ne soient mises en oeuvre les injections dans le cadre des essais cliniques. Ces études ont montré que les combinaisons bivalentes étaient équivalentes aux monovalents correspondants notamment en termes de pouvoir immunogène. Ainsi, la combinaison bivalente de SmithKline Beecham Biologicals a satisfait aux exigences des autorités de Grande-Bretagne et a déjà reçu une autorisation de mise sur le marché dans ce pays. Cette autorisation a néanmoins pour contrainte une date de péremption établie à 12 mois après fabrication.

Dans le domaine des vaccins, une date de péremption de 12 mois ne peut être considérée comme suffisante compte tenu notamment du temps nécessaire aux contrôles des lots avant distribution. Une date de péremption établie à 24 ou, mieux encore, 36 mois facilite grandement la commercialisation des lots.

Or, dans le cas des combinaisons HA-Vi, la demanderesse s'est aperçue que, au-delà de 16 18 mois passée la date de fabrication, la valence Vi perdait progressivement son pouvoir immunogène et a émis une hypothèse sur l'origine de cette instabilité. En effet, les groupements O-acétyls du polysaccharide Vi qui sont caractéristiques de l'immunogénicité du Vi, s'hydrolysent au cours du temps, surtout dans des conditions alcalines. Cette hydrolyse est tenue pour responsable de la diminution du pouvoir immunogène du polysaccharide Vi et elle serait due à l'adsorption du composant Vi sur l'hydroxyde d'aluminium qui est présent dans la combinaison bivalente en tant qu'adjuvant de la valence HA. La valence Vi s'adsorbe immédiatement sur le gel d'aluminium dès que les vaccins monovalents sont mélangés l'un à l'autre. Cette adsorption entraîne le maintien du polysaccharide Vi dans un environnement alcalin. En effet, l'hydroxyde d'aluminium étant chargé positivement, attire les ions OH- du milieu ce qui occasionne une augmentation du pH dans le micro-environnement du gel d'aluminium où se trouve le Vi suite à son adsorption. L'hydrolyse des O-acétyls quant à elle est un phénomène très lent dont les effets sont réellement perceptibles au bout de 16 - 18 mois environ.

Non seulement la demanderesse a mis en évidence le problème de l'instabilité de la valence Vi au cours du temps, mais elle propose une solution qui consiste à rajouter dans la combinaison bivalente un anion tel qu'un ion phosphate ou citrate qui empêche l'adsorption de la valence Vi sur le gel d'aluminium tout en maintenant la valence HA sous forme adjuvantée.

Dans son enseignement le plus général, l'invention porte donc sur une composition vaccinale comprenant au moins deux valences ; (i) une première valence qui est la valence Hépatite A et qui est adjuvantée avec de l'hydroxyde d'aluminium et (ii) une deuxième valence qui est la valence fièvre typhoïde constituée par le polysaccharide Vi de la capsule de Salmonella typhi qui contient un polysaccharide de capsule bactérienne comportant un ou des groupements O-acétyls et qui n'est pas adsorbée sur l'hydroxyde aluminium grâce auquel la première valence est adjuvantée, en raison de la présence d'un composé additionnel venant empêcher l'adsorption de la deuxième valence sur l'hydroxyde d'aluminium, sans perturber l'adsorption de la première valence.

Selon un mode de réalisation avantageux, l'adsorption de la deuxième valence est empêchée par la présence d'un composé anionique protecteur à condition qu'il présente toutes les garanties de sécurité nécessaires pour une utilisation à des fins vaccinales. Ce composé protecteur est un phosphate, un citrate ou encore un carbonate. Il est également possible d'utiliser une combinaison de différents anions, par exemple une combinaison d'ions phosphate et citrate. A titre indicatif, on précise que les ions phosphate peuvent être notamment apportés par une solution contenant du phosphate monopotassique, du phosphate disodique et du chlorure de sodium.

L'usage d'un composé protecteur permet notamment de stabiliser l'activité antigénique de la deuxième valence sur une longue période (24 mois ou plus), de préférence au cours d'une conservation à température normale de stockage ou plus élevée (*e.g*. 37°C). La stabilité de l'activité immunogénique peut être estimée par des techniques diverses en mesurant cette activité au moment où la composition est fabriquée puis en effectuant des mesures identiques au cours du temps, ou au moins à 24 mois passée la date de fabrication, et en comparant les résultats obtenus. Lorsque les données chiffrées ne sont pas établies comme étant statistiquement différentes entre elles, alors on doit considérer que l'activité immunogénique est stable. Le titrage de l'activité immunogénique d'un antigène peut notamment être mis en oeuvre par la technique ELISA, en usage courant dans le domaine des vaccins.

La valence Hépatite A peut être constituée par du virus HA inactivé.

La deuxième valence est par définition constituée par le polysaccharide Vi de la capsule de Salmonella typhi, conjugué ou non, qui contient au sein de son unité répétée un ou des groupements O-acetyls.

Par « polysaccharide de capsule bactérienne » on entend un polysaccharide constitué par l'enchaînement de l'unité répétée caractéristique d'un polysaccharide de capsule, quelle que soit sa taille et indépendamment de modification annexe. L'unité répétée d'un polysaccharide entrant dans la composition selon l'invention, comporte nécessairement au moins un groupement O-acétyl.

Aux fins de la présente invention, le polysaccharide peut être obtenu sous forme purifiée à partir de la bactérie d'origine selon des techniques tout à fait conventionnelles. Selon les besoins, le polysaccharide peut être (i) fragmenté ou non et (ii) conjugué ou non à un polypeptide porteur tel que l'anatoxine diphtérique ou tétanique.

Dans un cadre plus spécifique, l'invention a pour objet une composition vaccinale comprenant
(i) la valence HA adjuvantée avec de l'hydroxyde d'aluminium (ii) la valence fièvre typhoïde constituée par du polysaccharide Vi, composition dans laquelle la valence Vi n'est pas adsorbée sur l'hydroxyde d'aluminium, et (iii) des ions phosphate, citrate ou carbonate; en quantité suffisante pour empêcher l'adsorption de la valence fièvre typhoïde tout en maintenant la première valence sous forme adjuvantée

Aux fins de la présente invention, la première valence peut être adjuvantée soit par précipitation avec l'hydroxide d'aluminium, soit par adsorption sur l'hydroxyde d'aluminium.

L'hydroxyde d'aluminium servant à adjuvanter la première valence peut être de l'aluminium hydroxyde pur (c'est-à-dire un composé d'aluminium ne comportant que des ions Al3+ et des ions hydroxydes) ou n'importe quel hydroxyde d'aluminium connu sous ce nom même si du point de vue chimique ils ne sont pas constitués exclusivement d'hydroxyde d'aluminium. Ainsi, il peut aussi s'agir de composés d'aluminium mixtes tels que ceux désignés sous le nom d'aluminium hydroxy phosphate ou hydroxy sulfate. De manière générale, il peut s'agir de tout composé d'aluminium comportant notamment des ions hydroxydes. A titre d'illustration, on cite l'hydroxyde d'aluminium Alhydrogel^{™} commercialisé par la Société Superfos Biosector.

Les ions phosphate, citrate ou carbonate (composé protecteur) doivent être ajoutés en quantité suffisante pour empêcher l'adsorption de la deuxième valence tout en maintenant la première valence sous forme adjuvantée. Cette quantité dépend de divers facteurs parmi lesquels, la quantité et la nature de la première valence, son mode d'adjuvantation, la quantité et la nature de l'hydroxyde d'aluminium et la quantité de la deuxième valence. L'homme de l'art dans le domaine des vaccins est tout à fait à même de prendre en compte ces contraintes afin de déterminer la quantité appropriée du composé empêchant l'adsorption de la deuxième valence une fois que les autres facteurs ont été établis, de telle sorte que l'hydroxyde d'aluminium soit saturé par les ions phosphate tout en maintenant la première valence sous forme adjuvantée.

Néanmoins, on précise que les vaccins du commerce contiennent de manière usuelle de 0,6 à 1,5 mg d'aluminium / ml. Dans les vaccins HepA du commerce, le gel d'alumine, présent aux doses conventionnelles (exprimé en quantité d'aluminium), se retrouve largement en excès par rapport à l'antigène HepA dans la mesure où les sites d'adsorption du gel d'alumine sont loin d'être saturés. Ainsi, en pratique, seule la quantité d'aluminium apparaît comme déterminante pour établir la quantité de composé protecteur qui doit se trouver présente.

Pour une composition selon l'invention contenant 0,3 mg d'aluminium sous forme d'hydroxyde d'aluminium et sous un volume de 0,5 ml, il convient de rajouter environ 20 mM d'ions phosphate. Si la dose d'aluminium est doublée sous ce même volume, il convient de rajouter deux fois plus d'ions phosphate, soit 40 mM. Mais pour une composition selon l'invention contenant 0,6 mg d'aluminium sous un volume de 1 ml, 20 mM d'ions phosphate suffisent.

A titre d'illustration, on indique qu'un vaccin bivalent selon l'invention peut contenir sous un volume de 0,5 ml, (i) 160 unités antigéniques ou 1440 unités ELISA du virus de l'hépatite A inactivé adsorbées sur (ii) de l'hydroxyde d'aluminium contenant 0,3 mg d'aluminium ; (iii) 0,025 mg de polysaccharide Vi ; et (iv) 20 mM d'ions phosphate.

Les unités antigéniques et les unités ELISA citées ci-dessus sont de manière respective, des unités établies à l'aide de tests ELISA de référence propres aux sociétés Aventis Pasteur et SmithKline Beecham Biologicals (van Hoecke et al, J. Travel. Med. (1998) 5 : 116 et André et al, in Prog. Med. Virol., Melnick JL Ed, Basel, Karger (1990) 37 : 72). Il ne peut en être autrement puisqu'il n'existe pas de référence standardisée internationale pour le vaccin HepA.

Une composition selon l'invention est avantageusement sous forme liquide et une dose vaccinale est avantageusement formulée sous un volume compris entre 0,5 et 1 ml, bornes incluses.

Une composition selon l'invention peut être réalisée :
(i) en ajoutant des ions phosphate, citrate ou carbonate à une préparation contenant une valence autre que la valence fièvre typhoïde, adjuvantée par un hydroxyde d'aluminium ; et
(ii) en mélangeant la préparation obtenue au point (i) à une préparation contenant la valence fièvre typhoïde; procédé dans lequel la première valence est l'hépatite A, la valence fièvre typhoïde est constituée par le polysaccharide Vi de la Salmonella typhi, et les ions phosphate, citrate ou carbonate sont ajoutés en quantité suffisante pour empêcher l'adsorption de la deuxième valence tout en maintenant la première valence sous forme adjuvantée.

### Exemple : Préparation d'une composition HA Vi selon l'invention

### A - Préparation du composant HA adsorbé

99 ml d'un lot de virus HA inactivé dont le titre antigénique est de 885 U ELISA/ml est mélangé à 5,94 ml de 2-phenoxy-éthanol à 25 %. L'homogénisation est poursuivie pendant 15 min puis la préparation est filtrée sur filtre Millipak 40 MPGL 04SH2. Après filtration on obtient 95 ml d'une préparation titrant à 835 U ELISA/ml.

On ajoute à cette préparation 47,5 ml d'un gel d'alumine contenant 3,14 mg d'Aluminium / ml. On complète par 48 ml de PBS (tampon phosphate) 40 mM. L'agitation est poursuivie une nuit à 5°C (18 hrs) afin que le composant HA s'adsorbe sur le gel d'alumine. Le pH est de 7,28.

### B - Préparation d'une solution de polysaccharide Vi 10 fois concentrée

On prépare une poudre de polysaccharide Vi selon la méthode de Gotschlich et al, Prog. Immunobiol. Standard (1972) 5 : 485. Dans un flacon de 30 ml contenant 16,64 mg de Vi (9,5 g% d'humidité résiduelle, soit 15,06 mg de poids sec), on verse petit à petit 25,1 ml d'eau distillée préparée pour injection (ppi), sous agitation continue. On laisse l'agitation se poursuivre 24 hrs à 5°C afin d'obtenir la dissolution complète du polysaccharide. Cette préparation est filtrée sur filtre Millex 0,22 µm GV SLGV 025. Le filtre est rincé avec 5 ml d'eau distillée ppi. Le volume final est donc de 30,1 ml.

### C - Préparation du vaccin bivalent HAVi

A 62 ml de la préparation obtenue au point A (HA), on ajoute 10,6 ml d'une solution de phosphate 94 mM, puis 8,1 ml de la préparation obtenue au point B (Vi). Le caractéristiques du vaccin ainsi obtenu sont les suivantes :
Concentration finale en phosphate : 20 mM
pH : 7,3
Osmolarité : 578 mosm / kg

Cette préparation, appelée préparation P2, est ensuite répartie en dose de 0,5 ml.

En parallèle on a aussi préparé deux autres compositions bivalentes P2' et P2" contenant non plus 20 mM de phosphate mais de manière respective, 10 et 40 mM de phosphate. Pour ce faire, on a rajouté 10,6 ml d'une solution de phosphate à 17,6 mM ou 246 mM selon l'espèce.

Le comportement des composants HA et Vi dans les compositions P2, P2' et P2" a immédiatement été étudié en dosant ces composants par ELISA dans les compositions telles quelles ou après centrifugation. On a constaté qu'à 10 mM de phosphate, le HA restait adsorbé mais Vi s'adsorbait sur le gel d'alumine tandis qu'à 40 mM de phosphate le Vi ne s'adsorbait plus mais le HA se désorbait partiellement. A 20 mM de phosphate, les conditions recherchées sont remplies : le HA reste adsorbé tandis que le Vi ne s'adsorbe pas.

Par la suite, la stabilité des doses P2 a été étudiée à 5°C ± 3°C pendant 30 mois. On a notamment évalué par ELISA, la capacité antigénique du polysaccharide Vi dans les doses vaccinales prises dans leur intégralité et dans le surnageant après centrifugation (le composant adsorbé sur le gel d'alumine sédimente avec le gel) ; cela permet en outre d'établir le pourcentage de Vi non adsorbé.

L'ELISA est mis en oeuvre en méthode indirecte. L'antigène Vi à doser est pris en sandwich entre des anticorps anti-Vi tapissant le fond d'une plaque et des anticorps anti-Vi de souris. On rajoute un anticorps anti IgG de souris biotinylé, puis le complexe streptavidine biotinylée couplée à la peroxydase du raifort. La réaction est révélée par ajout du substrat Ortho Phenylène diamine Dihydrochloride (OPD). La dégradation de l'OPD se traduit par une coloration brune orangée proportionnelle à la quantité d'antigène Vi. Son intensité est mesurée au spectrophotomètre.

Dans des plaques de 96 puits on répartit 100 µl d'un sérum anti *Salmonella typhi.* On laisse incuber 5 hrs à 37°C puis on vide la plaque et on effectue 3 lavages en PBS (tampon phosphate) Tween 0.05 %. On sature les sites libres en ajoutant de 200 µl d'une solution de poudre de lait diluée en PBS. On incube 1 hr 30 à 37°C puis on vide la plaque et on effectue 3 lavages. On prépare des dilutions en série de raison 2 d'un vaccin étalon pour obtenir une gamme étalon. Les doses de vaccin à titrer ainsi qu'une dose de référence (permet de vérifier la gamme d'étalonnage) sont diluées de manière appropriée ; 100 µl de chaque dilution sont répartis dans les cupules et on laisse incuber une nuit à 37°C. On vide la plaque et procède à des lavages. On ajoute ensuite 100 µl par cupules d'un sérum de souris anti-Vi dilué de manière appropriée. On laisse incuber 1 hr à 37°C puis on vide la plaque et procède à des lavages. On fixe alors les anti-immunoglobines de souris biotinylées (100 µl par cupules d'une dilution appropriée). On laisse incuber 1 hr à 37°C puis on vide la plaque et procède à des lavages. On fixe alors la streptavidine biotinylée couplée à la peroxidase (100 µl par cupules d'une dilution appropriée). On laisse incuber 1 hr à 37°C puis on vide la plaque et procède à des lavages. On révèle la plaque en ajoutant 100 µl par cupules d'une solution d'OPD à 1 mg/ml en tampon citrate phosphate pH 5. On laisse incuber 30 min à température ambiante dans l'obscurité avant d'ajouter 100 µl d'acide sulfurique 2 N dans les cupules. La lecture de la plaque est effectuée au spectrophotomètre à 492 nm. On établit la courbe étalon de l'adsorbance en fonction de la concentration. Le titre de chacune des dilutions testées est calculé par rapport à la courbe étalon et est exprimé en ng/ml. Pour obtenir le titre moyen de chaque dose vaccinale testée on fait la moyenne des titres obtenus avec l'ensemble des dilutions. Le titre est rendu en µg/dose.

On a également mesuré la quantité des O-acétyls du polysaccharide Vi présent dans le surnageant, après centrifugation. Les O-acétyls sont titrés par une méthode colorimétrique utilisant l'hydroxylamine (Hestrin S. J. Biol. Chim. (1949) 180 : 249). L'hydroxylamine en milieu alcalin forme avec les esters un acide hydroxamique qui en présence de sel ferrique donne une coloration marron dont l'intensité est mesurée au spectrophotomètre à 540 nm.

On a mené en parallèle une étude identique avec une préparation dite préparation P1, préparée de la même manière que P2, à la seule différence que l'on ne rajoute pas de phosphate au moment de la constitution du vaccin bivalent. Dans ce cas, le composant Vi s'adsorbe immédiatement sur le gel d'alumine et afin de réaliser son dosage après centrifugation, comme pour P2, il convient de le désorber au préalable. Cette désorption est obtenue par modification du pH et de la force ionique du milieu. Après centrifugation, le gel d'alumine est mis en contact avec une solution de citrate trisodique 150 mM pendant 6 hrs à 37°C. Puis on centrifuge pour recueillir le surnageant dans lequel se retrouve le composant Vi.

Les résultats sont présentés dans le Tableau I ci-après.

**Tableau I**

| **5°C** | | | T0 | 3 mois | 6 mois | 9 mois | 12 mois | 18 mois | 24 mois | 30 mois |
|---|---|---|---|---|---|---|---|---|---|---|
| Vi dans le vaccin entier | Vi ELISA (µg/dose) | P2 | 23,6 | 25,5 | 21,7 | 21,3 | 18,5 | 23,7 | 24,3 | 26,5 |
| | | *P1* | *24,4* | *23,3* | *22,2* | *19,8* | *17,9* | *20,5* | *18,6* | 19, 3 |
| Vi dans le surnageant après centrifugation | Vi ELISA (µg/dose) | P2 | 24,3 | 26 | 23,1 | 21,8 | 17,9 | 25 | 21,9 | 25,8 |
| | | *P1* | *21* | *16,9* | *16,5* | *15,4* | *12* | *14,7* | *21,8* | *15,7* |
| | O-acétyls (µmole/dose) | P2 | 0,127 | 0,148 | 0,116 | 0,117 | 0,130 | 0,134 | 0,095 | 0,143 |
| | | *P1* | *0,081* | *0,056* | *0,055* | *0,055* | *0,061* | *0,057* | *0,090* | *0,054* |
| | Polysaccharides (µg/dose) | P2 | 32,5 | 30,5 | 27,6 | 31 | 30,4 | 29,1 | 31,7 | 29,3 |
| | | *P1* | *20,8* | *22* | *15,6* | *16,2* | *18,5* | *16,5* | *17* | *18,4* |
| % de désorption du Vi dans *P1* | | | *86%* | *72%* | *74%* | *78%* | *67%* | *72%* | *63%* | *81,6%* |

La stabilité de la formulation P2 a aussi été étudiée à 25°C ± 2°C pendant 6 mois et à 37°C ± 3°C pendant 3 mois. Les résultats sont présentés dans les Tableaux II et III ci-après.

**Tableau II**

| **25°C** | | | T0 | 1 mois | 3 mois | 6 mois |
|---|---|---|---|---|---|---|
| Vi dans le vaccin entier | Vi ELISA (µg/dose) | P2 | 23,6 | 23,6 | 21,7 | 20,6 |
| | | *P1* | *24,4* | *19,6* | *11,3* | *7,4* |
| Vi dans le surnageant après centrifugation | Vi ELISA (µg/dose) | P2 | 24,3 | 25,5 | 22,6 | 20,1 |
| | | *P1* | *21* | *15* | *10,3* | *6,3* |
| | O-acétyls (µmole/dose) | P2 | 0,127 | 0,130 | 0,103 | 0,137 |
| | | *P1* | *0,081* | *0,053* | *0,041* | *0,029* |
| | Polysaccharides (µg/dose) | P2 | 32,5 | 31,6 | 25,5 | 27,2 |
| | | *P1* | *20,8* | *17,3* | *10,3* | *6,3* |
| % de désorption du Vi dans *P1* | | | *86%* | *77%* | *91%* | *85%* |

**Tableau III**

| **37°C** | | | T0 | 1 mois | 3 mois |
|---|---|---|---|---|---|
| Vi dans le vaccin entier | Vi ELISA (µg/dose) | P2 | 23,6 | 24,1 | 21 |
| | | *P1* | *24,4* | *12,7* | *5,6* |
| Vi dans le surnageant après centrifugation | Vi ELISA (µg/dose) | P2 | 24,3 | 25,5 | 21,1 |
| | | *P1* | *21* | *9,9* | *4,7* |
| | O-acétyls (µmole/dose) | P2 | 0,127 | 0,143 | 0,104 |
| | | *P1* | *0,081* | *0,050* | *0,026* |
| | Polysaccharides (µg/dose) | P2 | 32,5 | 36 | 22,5 |
| | | *P1* | *20,8* | *15,4* | *9,9* |
| % de désorption du Vi dans *P1* | | | *86%* | *78%* | *84%* |

## Revendications

1. Une composition vaccinale comprenant (i) une première valence adjuvantée avec un composé d'aluminium comportant des ions hydroxydes, (ii) une deuxième valence qui contient un polysaccharide de capsule bactérienne comportant un ou des groupements O-acétyles et (iii) des ions phosphate, citrate ou carbonate en quantité suffisante pour empêcher l'adsorption de la deuxième valence tout en maintenant la première valence sous forme adjuvantée ; composition dans laquelle la première valence est la valence hépatite A et la deuxième valence est la valence fièvre typhoïde qui est constituée par le polysaccharide Vi de la capsule de *Salmonella typhi.*

2. Une composition vaccinale selon la revendication 1, dans laquelle la valence hépatite A est constituée par du virus de l'hépatite A inactivé.

3. Une composition vaccinale selon la revendication 2, dans laquelle la valence fièvre typhoïde possède un titre antigénique stable pendant au moins 24 mois.

4. Une composition selon l'une des revendications 1 à 3, dans laquelle la première valence est adjuvantée par un composé d'aluminium qui est de l'aluminium hydroxyde ou de l'aluminium hydroxy phosphate.

5. Une composition selon l'une des revendications 1 à 4, dans laquelle la première valence est adjuvantée par adsorption sur un composé d'aluminium.

6. Un procédé de fabrication d'une composition vaccinale selon l'une des revendications 1 à 5, selon lequel :
(i) on ajoute des ions phosphate, citrate ou carbonate, à une préparation contenant une première valence adjuvantée par un composé d'aluminium comportant des ions hydroxydes ; et
(ii) on mélange la préparation obtenue au point (i) à une préparation contenant une deuxième valence constituée par un polysaccharide de capsule bactérienne comportant un ou des groupements O-acétyles ; procédé dans lequel la première valence est l'hépatite A, la deuxième valence est la valence fièvre typhoïde qui est constituée par le polysaccharide Vi de la capsule de *Salmonella typhi* ; et les ions phosphate, citrate ou carbonate sont ajoutés en quantité suffisante pour empêcher l'adsorption de la deuxième valence tout en maintenant la première valence sous forme adjuvantée.

## Claims

1. A vaccine composition comprising (i) a first valence adjuvanted with an aluminum compound comprising hydroxide ions, (ii) a second valence which contains a bacterial capsule polysaccharide comprising one or more O-acetyl groups and (iii) phosphate, citrate or carbonate ions in a sufficient amount to prevent adsorption of the second valence while maintaining the first valence in an adjuvanted form ; in which the first valence is the hepatitis A valence and the second valence is the typhoid fever valence which consists of *Salmonella typhi* capsular Vi polysaccharide..

2. A vaccine composition as claimed in claim 1, in which the hepatitis A valence consists of inactivated hepatitis A virus.

3. A vaccine composition as claimed in claim 2, in which the typhoid fever valence has a stable antigenic titer for at least 24 months.

4. A composition as claimed in one of claims 1 to 3, in which the first valence is adjuvanted with an aluminum compound which is aluminum hydroxide or aluminum hydroxyphosphate.

5. A composition as claimed in one of claims 1 to 4, in which the first valence is adjuvanted by adsorption onto an aluminum compound.

6. A method for manufacturing an immunization composition as claimed in one of claims 1 to 5, according to which:
(i) phosphate, citrate or carbonate ions are added to a preparation containing a first valence adjuvanted with an aluminum compound comprising hydroxide ions;
(ii) the preparation obtained in point (i) is mixed with a preparation containing a second valence consisting of a bacterial capsular polysaccharide comprising one or more O-acetyl groups ;
in which the first valence is the hepatitis A valence and the second valence is the typhoid fever valence which consists of *Salmonella typhi* capsular Vi polysaccharide ; and the phosphate, citrate or carbonate ions are added in a sufficient amount to prevent adsorption of the second valence while maintaining the first valence in an adjuvanted form.

## Patentansprüche

1. Vakzinzusammensetzung, umfassend (i) eine erste Valenz, die mit einer Aluminiumverbindung, die Hydroxidionen enthält, als Adjuvans versetzt ist, (ii) eine zweite Valenz, die ein Bakterienkapsel-Polysaccharid, das eine oder mehrere 0-Acetylgruppierung(en) enthält, enthält, und (iii) Phosphat-, Citrat- oder Carbonationen in der ausreichenden Menge, um die Adsorption der zweiter Valenz zu hindern, die erste Valenz in einer adjuvander Form aufrechterhaltend; Zusammensetzung in der die erste Valenz die Hepatitis A-Valenz ist und in der die zweite Valenz die Flecktyphus-Valenz ist, die aus dem Polysaccharid Vi der Kepsel von *Salmonella typhi* besteht.

2. Vakzinzusammensetzung nach Anspruch 1, in der die Hepatitis-A-Valenz aus dem inaktivierten Hepatitis A-Virus Besteht.

3. Vakzinzusammensetzung nach Anspruch 2, in der die Flecktyphus-Valenz während wenigstens 24 Monaten einen stabilen Antigentiter besitzt.

4. Zusammensetzung nach einem der Ansprüche 1 bis 3, in der die erste Valenz mit einer Aluminiumverbindung als adjuvans versetzt ist, die aluminiumhydroxid oder Aluminiumhydroxidphostphat ist.

5. Zusammensetzung nach einem der Ansprüche 1 bis 4, in der die erste Valenz mit einer Adsorption an einer Aluminiumverbingung als Adjuvans verbunden ist.

6. Verfahren zur Herstellung einer Vakzinzusammensetzung nach einem der Ansprüche 1 bis 5, wobei
(i) man phosphat-, citrat- oder carbonationen einer Präparation zusetzt, die eine erste Valenz, die mit einer Aluminiumverbingung, die Hydroxidionen enthält, als Adjuvans versetzt ist, enthält; und
(ii) man die gemass Abschnitt (i) erhaltene Präparation einer Präparation zumischt, die eine zweite Valenz enthält,welche aus einem Bakterienkapsel-Polysaccharid, das eine oder mehere O-Acetylgruppierung(en) enthält, besteht;
Verfahren in dem die erste Valenz die Hepatitis A-Valenz ist und in der die zweite Valenz die Flecktyphus-Valenz ist, die aus dem Polysaccharid Vi der Kepsel von *Salmonella typhi* besteht; und die phosphat-, citrat- oder carbonationen sind in der ausreichenden Menge zusetzt, um die Adsorption der zweiter Valenz zu hindern, die erste Valenz in einer adjuvander Form aufrechterhaltend.
